# FASCICULE DE BREVET EUROPEEN

(11) **EP 3 320 466 B1**
(45) Date de publication et mention de la délivrance du brevet: **25.09.2024**
(21) Numéro de dépôt: 16745636.7
(22) Date de dépôt: 11.07.2016
(51) Int. Cl.: G16H 20/40, G16H 40/63, A61M 16/00

(54) **DISPOSITIF DE STIMULATION DE L`AIR TRACHÉO-BRONCHIQUE**
VORRICHTUNG ZUR STIMULIERUNG DER TRACHEOBRONCHIALLUFT
DEVICE FOR STIMULATING TRACHEOBRONCHIAL AIR

(30) Priorité: 10.07.2015 FR 1556616
(43) Date de publication de la demande: 16.05.2018
(73) Titulaire: Physio-Assist, 13593 Aix-en-Provence (FR)
(72) Inventeur: Lantz, Jean-Sébastien, 13100 Aix-en-Provence (FR); Mithalal, Adrien, 34090 Montpellier (FR)
(74) Mandataire: Icosa
(86) Numéro de dépôt international: PCT/EP2016/066453
(87) Numéro de publication internationale: WO 2017/009299

(56) Documents cités:
- WO-A1-96/35468
- WO-A2-2010/058308
- US-A- 2 918 917
- US-A1- 2005 051 174
- US-A1- 2012 285 460

## Description

### DOMAINE TECHNIQUE DE L'INVENTION

La présente invention concerne le domaine du traitement des troubles ventilatoires obstructifs et, plus spécifiquement, un dispositif de stimulation du mucus en vue d'améliorer son expectoration.

### ARRIERE-PLAN TECHNOLOGIQUE DE L'INVENTION

Chez l'individu sain, les poumons sont recouverts par un « film » que l'on appelle mucus, dont l'épaisseur est de quelques millimètres et la consistance normale est très fluide. La fonction de ce mucus correspond à la protection des cellules pulmonaires en les isolants d'un contact direct avec l'air inspiré par les poumons. Le renouvellement de ce mucus est assuré par des cils, excroissances mobiles, se trouvant à la surface des bronches. Ces cils, qualifiés de vibratiles, battent vers la partie proximale des voies aériennes, éliminant les particules inhalées, emprisonnées dans la phase gel du mucus, lesquelles particules glissent sur la couche de cils, à la manière d'un tapis roulant. La clairance (la capacité d'un tissu, organe ou organisme à éliminer d'un fluide une substance donnée) mucociliaire ainsi définie, s'effectue à une vitesse de l'ordre de 5 mm/min dans la trachée, assurant le renouvellement de la couche de mucus toutes les 20 min environ. Cette clairance mucociliaire permet ainsi d'éliminer le mucus vers le pharynx où il sera dégluti ou expectoré.

Maintenant, il existe différentes pathologies résultant en un trouble ventilatoire obstructif, ou syndrome obstructif pulmonaire, qui se caractérise par une accumulation de mucus et se traduit par une limitation des débits dans l'arbre bronchique et par une augmentation des résistances aériennes.

Plus généralement, ce trouble ventilatoire obstructif constitue la résultante d'une affection chronique correspondant à une bronchectasie (appelée également bronchiectasie ou encore dilatation des bronches (DDB)), le plus souvent acquise à la suite d'une maladie des bronches, du poumon ou de la plèvre. Cette bronchectasie, qui peut être localisée ou diffuse, se caractérise par une dilatation des bronches de petit et moyen calibre et s'accompagne souvent d'une expectoration muco-purulente abondante, qui traduit l'infection surajoutée.

Les causes possibles de bronchectasies sont multiples et regroupent notamment la mucoviscidose, les BPCO (p. ex. emphysèmes pulmonaires ou bronchites chroniques), avec notamment les infections sévères de la petite enfance (p. ex. bronchiolite), les dyskinésies ciliaires (p. ex. syndrome de KARTAGENER) ou encore la sténose bronchique (par un corps étranger ou une tumeur), les séquelles de tuberculoses pulmonaires (cause la plus fréquente), ou les déficits congénitaux ou acquis en Ig (A, G, ou M).

En ce qui concerne la mucoviscidose, il s'agit d'une maladie génétique affectant les épithéliums glandulaires de nombreux organes. C'est la maladie génétique létale à transmission autosomique récessive la plus fréquente dans les populations de type caucasienne, alors qu'elle est très rare dans les populations africaines et asiatiques. Elle est liée à des mutations du gène CFTR sur le chromosome 7, entraînant une altération de la protéine CFTR (Cystic Fibrosis Transmembrane conductance Regulator) qui est un canal ionique perméable au chlore et dont la fonction est de réguler le transport du chlore à travers les membranes cellulaires. Cette altération entraîne une augmentation de la viscosité du mucus et son accumulation dans les voies respiratoires et digestives. La maladie touche de nombreux organes mais les atteintes respiratoires sont prédominantes et représentent l'essentiel de la morbidité. La forme clinique la plus fréquente associe troubles respiratoires, troubles digestifs et troubles de la croissance staturopondérale. Il n'y a pas de traitement curatif mais les progrès de la prise en charge ont permis d'améliorer la qualité et l'espérance de vie des patients ; ainsi en France, l'espérance de vie à la naissance est passée de 7 ans en 1965 à 47 ans en 2005.

L'étiologie de la BPCO ou Broncho Pneumopathie Obstructive chronique est bien différente de la mucoviscidose puisque sa cause principale en est le tabagisme. Cette maladie se caractérise, au niveau respiratoire, par une obstruction lente et progressive des voies aériennes et des poumons, associée à une distension permanente des alvéoles pulmonaires avec destruction des parois alvéolaires. La BPCO correspond principalement à la bronchite chronique (p. ex. bronchiolite) ou à l'emphysème pulmonaire et c'est d'ailleurs parce qu'il est rare qu'un patient souffre d'emphysème pur ou de bronchite chronique pure que le terme de BPCO est apparu. Chez le patient atteint de BPCO, le métabolisme anaérobie se retrouve préférentiellement sollicité, au détriment du métabolisme aérobie. L'entretien et la restauration du fonctionnement du métabolisme aérobie apparaissent aujourd'hui comme les enjeux majeurs de réadaptation en faveur de la qualité de vie des patients souffrant de BPCO.

Autre cause de bronchectasie, la dyskinésie ciliaire primitive (DCP) (appelée également syndrome de KARTAGENER ou de SIEWERT) est, comme la mucoviscidose, une maladie génétique portant atteinte au système respiratoire. Le terme dyskinésie qualifie le défaut de mouvement ciliaire observé car cette maladie touche les cils vibratiles de l'organisme. Maintenant, outre la dyskinésie ciliaire primitive, et observée dès la naissance, il existe une dyskinésie ciliaire secondaire (DCS), diagnostiquée ultérieurement. La DCP n'est pas une maladie contagieuse, mais des infections pulmonaires secondaires à la maladie peuvent se développer. Aussi, il est conseillé d'être prudent dans le contact de patients DCP avec les patients sensibles (autres DCP, MUCO, Immunodéprimé,...).

Maintenant, et pour tous ces troubles respiratoires obstructifs, le patient présente une accumulation de mucus bronchique qui, de par sa stagnation, est responsable d'infections pouvant conduire à de graves complications pulmonaires. Aussi, est t'il important de procéder à un désencombrement régulier chez ces patients.

Pendant longtemps, on a donc tenté d'utiliser des mucolytiques ou des mucorégulateurs. Malheureusement, ces derniers n'ont montré qu'un faible service médical rendu et les traitements utilisés aujourd'hui se limitent donc le plus souvent à l'administration de bronchodilatateurs et à des séances de kinésithérapies respiratoires. Ce traitement s'apparente en fait à une « toilette bronchique », dont le but est d'éviter la surinfection dont sont l'objet le plus souvent ces patients.

Cependant cette toilette bronchique peut s'avérer traumatisante pour le patient et reste d'une efficacité limitée lorsque le mucus est trop visqueux ou élastique.

Aussi, et en vue d'assister les patients dans cette toilette bronchique, on connaît, dans l'état de la technique, différents dispositifs destinés à faciliter l'expulsion de mucus ont été développés.

On connait ainsi un dispositif utilisant l'IPV^{®} (Intrapulmonary Percussive Ventilation) inventé dans les années 1980 par le docteur Forrest BIRD. Ce dispositif passe par l'application d'un masque respiratoire et l'administration d'air au patient sous la forme de puissantes pulsations d'air saccadées de sorte de décrocher le mucus des bronches et de faciliter son expectoration par le patient. De tels dispositifs incluent notamment le PERCUSIONATOR^{®}. D'une utilisation très « traumatique », ce dispositif est aujourd'hui peu utilisé.

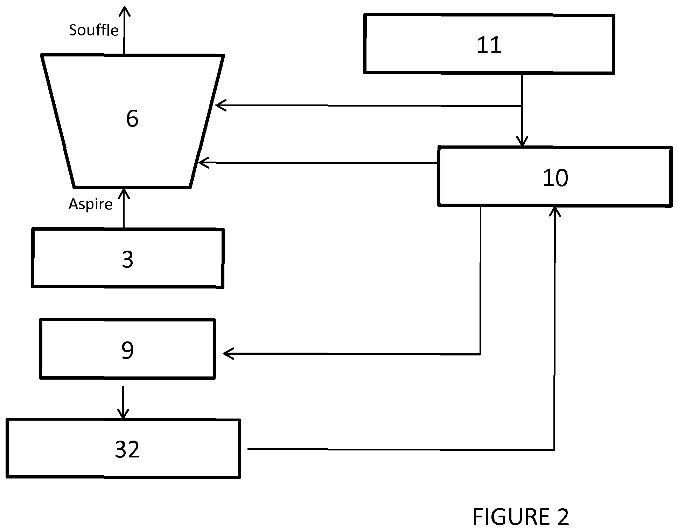

On connait également un autre dispositif, décrit dans le brevet FR 2 733 917, visant à stimuler l'air trachéo-bronchique d'un patient, de manière intra-pulmonaire, de telle manière à fluidifier le mucus bronchique. Ce document enseigne que l'air inspiré doit vibrer et que lors de l'expiration, il faut susciter la toux du patient en superposant des dépressions de faible amplitude et de courte durée pour ne pas induire de collapsus des parois bronchiques. En effet, la figure 2 du document montre que l'amplitude des faibles dépressions ne dépasse pas 10 mbars et que la fréquence des dépressions est de l'ordre de 5 Hz, car d'après le diagramme, on observe une dizaine de dépressions par expiration et la durée de l'expiration étant d'environ 2 secondes chez l'adulte au repos on en déduit la fréquence citée ci-dessus. Ainsi, ce document enseigne d'une part de provoquer la toux ce qui génère un désagrément chez le patient et d'autre part que les impulsions sont de faibles amplitudes de l'ordre de 10 mbars et de basses fréquences de l'ordre de 5 Hz pour éviter le collapsus bronchique. Nous considérons ce document comme étant l'état de la technique le plus proche.

On connaît également des documents ayant le même inconvénient de générer des dépressions pendant l'inspiration et ayant pour but de provoquer la toux.

Ainsi, la demande de brevet US 2009/126734 enseigne un dispositif visant à stimuler l'air trachéo-bronchique d'un patient, de manière intra-pulmonaire en utilisant une source de gaz pressurisé délivrant des pressions positives à l'aide d'une vanne pour constituer des impulsions percussives de gaz et deux capteurs, l'un mesurant la pression dans l'interface du patient et l'autre mesurant la pression de la ligne de sortie. Ainsi, lesdits capteurs permettent de fournir un retour sur la pression de l'air inspiré et expiré permettant de déterminer au mieux la fréquence des impulsions pour le patient. Une interface graphique permet d'afficher les paramètres opérationnels et de communiquer avec le contrôleur qui gère le dispositif et mémorise un ou plusieurs protocoles thérapeutiques permettant de générer une première fréquence pendant un premier intervalle puis une seconde fréquence.

La demande de brevet US 2012285460 enseigne un Appareil de MIE (Mechanical Inspiration Expiration) possédant un ventilateur, une vanne de direction, un oscillateur, et un connecteur de tuyau masque.

Le ventilateur est relié à la soupape de direction, qui est relié à l'oscillateur, qui est relié au connecteur de tuyau. Au cours de l'inspiration, une soupape de direction relie l'échappement d'un ventilateur générant une pression positive à un oscillateur, et au niveau du connecteur de tuyau. Pendant expiration, la soupape de direction relie l'admission du ventilateur pour provoquer une pression négative au niveau du connecteur de tuyau et de l'oscillateur. L'oscillateur est une vanne papillon avec un disque rotatif de 360 °. Au cours de l'inspiration, le disque module le flux d'air. Pendant l'expiration, l'oscillateur est inactif ou en mode battement. Lorsqu'il est inactif, le disque est fixe pour permettre l'écoulement maximal de l'air. En mode battement, le disque tourne de façon continue de sorte que le flux d'air alterne rapidement entre le maximum et le minimum de débit. Enfin ce document nécessite que l'utilisateur appuie sur le bouton expiration pendant le cycle d'expiration (figure 10).

La demande US 20050051174 enseigne un système amélioré d'inspiration - expiration assisté par des impulsions pour l'élimination des sécrétions broncho-pulmonaires qui comprend un conduit pour le raccordement aux voies respiratoires du patient, une source de pression qui fournit par le conduit des variations de pression alternativement positive et négative à une première fréquence correspondant à l'inspiration - expiration du patient et un mécanisme de commande permettant la variation de pression au cours des variations de pression positives et négatives à une seconde fréquence supérieure pour diminuer périodiquement la pression positive pendant les variations de pression positive et diminuer la pression négative pendant les variations de pression négative afin de fournir des impulsions de percussion au cours d'au moins une inspiration - expiration permettant d'éliminer les sécrétions broncho-pulmonaires des voies respiratoires du patient.

Ainsi, ce document enseigne des variations de pression lors de l'inspiration et lors de l'expiration du patient.

Par la demande WO2010/058308 il est enseigné de créer des cycles de pressions positives et négatives pendant les inspirations et expirations du patient.

Tous ces dispositifs génèrent des variations de pression pendant l'inspiration et ont pour but de provoquer une toux.

Ainsi, ces solutions présentent l'inconvénient de provoquer une expectoration, c'est-à-dire une toux, lors de l'expiration du patient. Ceci implique un risque de collapsus des parois bronchiques si la dépression n'est pas suffisamment faible en amplitude et de courte durée.

Dans ce contexte, il est intéressant de proposer une solution n'impliquant pas d'expectoration c'est-à-dire offrant la possibilité au patient de faire une expiration passive tout en améliorant la fluidification du mucus. En effet, la non implication des muscles respiratoires lors de l'expiration permet d'éviter le collapsus.

### DESCRIPTION GENERALE DE L'INVENTION

La présente invention a pour but de pallier certains inconvénients de l'art antérieur en proposant un dispositif de stimulation de l'air trachéo-bronchique d'un patient souffrant d'un trouble ventilatoire obstructif et apte à modifier la rhéologie de son mucus trachéo-bronchique. La présente invention est exposée dans le jeu de revendications joint.

### DESCRIPTION DES FIGURES ILLUSTRATIVES

D'autres particularités et avantages de la présente invention apparaîtront plus clairement à la lecture de la description ci-après, faite en référence aux dessins annexés, dans lesquels :

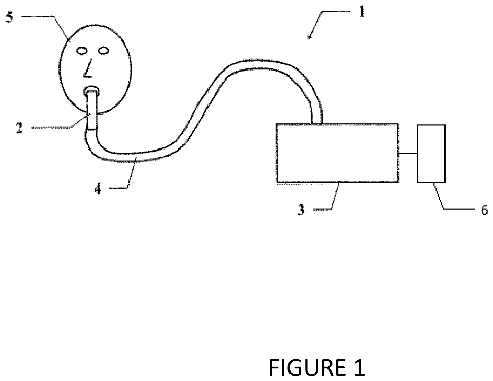

- la figure 1 représente le dispositif connecté au patient,
- la figure 2 représente un schéma du fonctionnement du dispositif,
- la figure 3 représente un exemple des signaux de dépression engendrés par le dispositif,

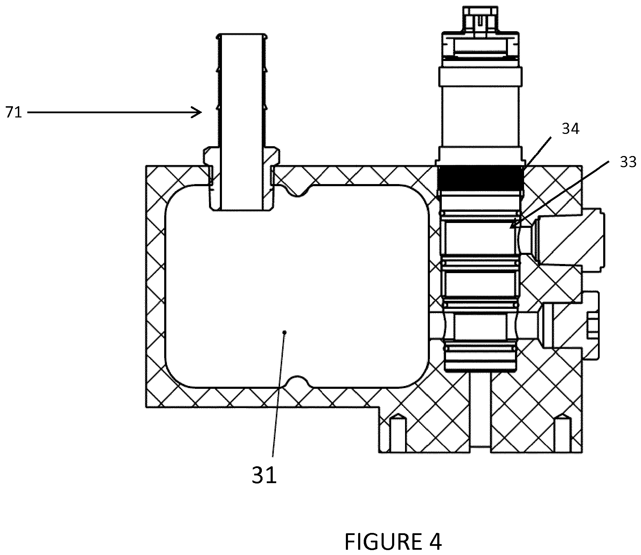

- la figure 4 représente une vue de coupe de l'embase,

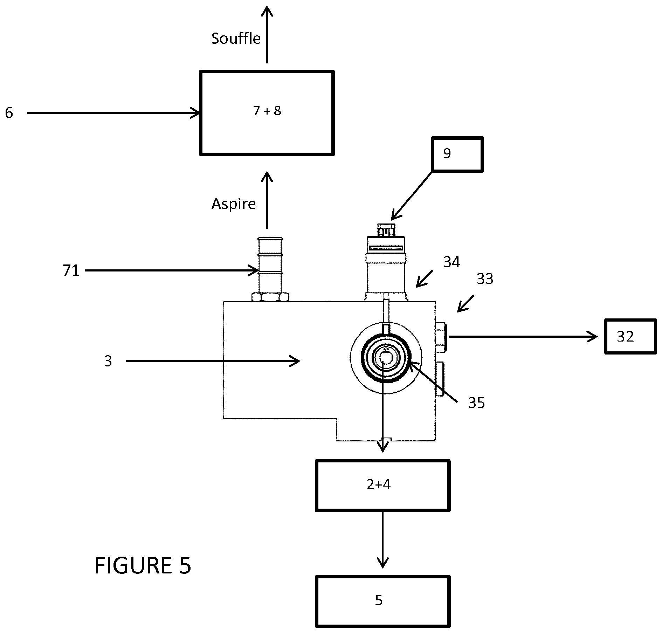

- la figure 5 représente une vue d'ensemble du système, avec l'embase de face.

### DESCRIPTION DES MODES DE REALISATION PREFERES DE L'INVENTION

La présente invention concerne un dispositif de stimulation (1) de l'air trachéo-bronchique d'un patient (5) souffrant d'un trouble ventilatoire obstructif et apte à modifier la rhéologie de son mucus trachéo-bronchique.

Au sens de la présente invention, l'expression « troubles ventilatoires obstructifs » regroupe les pathologies listées précédemment, mais s'étend également aux troubles associés à l'obstruction des voies nasales telle que la sinusite qui correspond à l'obstruction des sinus consécutive à une inflammation des muqueuses du nez résultant en un drainage altéré du mucus nasal.

Maintenant, et préférentiellement, le dispositif selon l'invention ciblera le mucus bronchique et visera donc à la stimulation de l'air intra-pulmonaire.

Le dispositif comporte un générateur de pression négative (6). Dans certains modes de réalisation, le générateur de pression négative (6) comprend une pompe à vide (7) avec un débit supérieur à 20 L/min et une capacité de descente en vide d'au moins 200 mbars. De préférence, ladite pompe à vide (7) est une pompe à membrane. De préférence, ladite pompe à vide (7) possède un débit supérieur à 40L/min et une capacité de descente en vide d'au moins 300 mbars. Dans certains modes de réalisation, ladite pompe à vide (7) est connectée sur sa sortie aspirante à un circuit de contrôle par l'intermédiaire d'un embout cannelé (71) et ladite pompe à vide (7) est connectée sur sa sortie soufflante à un piège à son (8). Ledit piège à son (8) est un système de cylindre capitonné qui a pour avantage d'atténuer le son produit lors du passage de l'air dans le système.

Le dispositif comporte une interface physiologique (2) apte à interfacer le dispositif avec l'appareil respiratoire du patient (5). Cette interface physiologique (2) comporte de préférence un embout buccal ou un masque respiratoire. De préférence, ladite interface physiologique est équipée d'une étiquette RFID pour le suivi du patient.

Le dispositif comporte un tuyau de raccordement (4) reliant l'interface physiologique (2) au générateur de pression négative (6). Dans un mode de réalisation, le tuyau de raccordement (4) relie l'interface physiologique (2) à une embase (3) pneumatique par la sortie (35) de ladite embase (3), ledit tuyau de raccordement (4) étant de préférence souple.

L'expiration passive réalisée avec le dispositif de la présente demande permet d'apporter une aide expiratoire, qui va augmenter le temps expiratoire. L'expiration est assurée par la machine, donc passive pour le patient, ce qui évite le collapsus bronchique physiologique qui est un obstacle majeur au désencombrement des voies aériennes. Cette expiration prolongée permet d'atteindre les voies aériennes périphériques, qui sont généralement la « cible » principale des manoeuvres de désencombrement.

Le dispositif comporte un circuit de contrôle capable, par un agencement particulier, de commander, pendant la phase d'expiration passive, l'application d'une succession d'alternances d'impulsions négatives de pression et de mise à l'atmosphère avec une fréquence déterminée, définissant un cycle, et d'au moins un rapport cyclique déterminé. On applique ces paramètres pendant une première partie d'un cycle d'expiration, puis on applique une deuxième fréquence et un deuxième rapport cyclique pendant une deuxième partie du cycle d'expiration, en réitérant un nombre défini de cycles d'expiration.

Ledit circuit de contrôle comporte de préférence une embase (3) pneumatique qui contient une chambre à vide (31), de préférence de 40 ml minimum. Ladite embase (3) pneumatique comporte de préférence un capteur de pression (32) qui est relié, via un tube, à un orifice (33) de l'embase (3) pneumatique. En outre, ledit capteur de pression (32) prend avantageusement la forme d'un capteur de pression relative mesurant la valeur de la dépression par rapport à la pression atmosphérique ambiante. Sur ce type de capteurs, les fluctuations de pression dues à des changements de météo ou d'altitude ont un impact direct sur la valeur mesurée. Si la pression qui s'exerce sur le capteur de pression relative est inférieure à la pression ambiante, on parle de pression relative négative ou, plus généralement de dépression, et la valeur est précédée d'un signe « - ».

Les capteurs de pression relative présentent une structure bien connue et ne comportent typiquement qu'un seul raccord de pression. La pression ambiante s'y exerce par une fente ou par un tube de mise à l'air libre situé à l'arrière de la membrane du capteur et cette mesure relative est ainsi compensée. L'invention étant en circuit fermé, et en dépression, un seul capteur suffit pour le dispositif. Les inventeurs ont pu mettre en évidence, qu'il importait pour optimiser l'efficacité du traitement et éviter la variabilité entre patients, que la dépression soit maintenue dans un intervalle spécifique à l'intérieur d'un cycle d'expiration. Cet intervalle de temps spécifique est désigné dans la présente demande sous le terme de "rapport cyclique" et définit le rapport entre la durée pendant laquelle la dépression est appliquée au patient et la durée totale d'un cycle. En d'autres termes, ce rapport cyclique correspond au temps pendant lequel les poumons du patient sont soumis à la dépression. Le contrôle de ce temps permet notamment d'éviter qu'il ne soit trop long et présente un risque de provoquer le collapsus des bronches. De plus, cette modulation du rapport cyclique de chaque dépression permet, au delà de la dépression générée qui a une action de cisaillement sur le mucus bronchique et qui fait donc chuter sa viscosité, d'appliquer un flux plus ou moins long, ce qui améliore le transport des sécrétions. Plus spécifiquement, il importe que la dépression appliquée au mucus trachéo-bronchique soit compris entre 40 et 100 millibars, de préférence entre 45 et 80 millibars, et de préférence encore entre 50 et 100 millibars. Ledit circuit de contrôle comporte également de préférence une électrovanne (9) qui a, de préférence, un diamètre d'ouverture inférieur à 6mm et un temps d'ouverture supérieur à 20ms. Ladite électrovanne (9) est reliée à ladite embase (3) pneumatique via un pas de visse (34).

Ledit circuit de contrôle a pour avantage de pouvoir faire varier le pouvoir liquéfiant du dispositif via un microcontrôleur (10) qui pilote la fréquence de commutation de l'électrovanne (9) et les temps d'ouverture et de fermeture de l'électrovanne (9). La première partie d'un cycle d'expiration comprend une fréquence de l'ordre de 10 à 15Hz et un rapport cyclique de 0,2 à 0,4 et la deuxième partie d'un cycle comprend une fréquence de l'ordre de 4 à 7 Hz et un rapport cyclique de l'ordre de 0,5 à 0,8. De préférence, la première partie d'un cycle d'expiration comprend une fréquence de 12Hz et un rapport cyclique de 0,3 et la deuxième partie d'un cycle comprend une fréquence de 6 Hz et un rapport cyclique de l'ordre de 0,6. De préférence, le nombre défini de cycle d'expiration est choisi au départ par l'opérateur ou le patient (5) et est adapté par l'appareil en fonction des résultats de durée de stimulation moyenne évaluées.

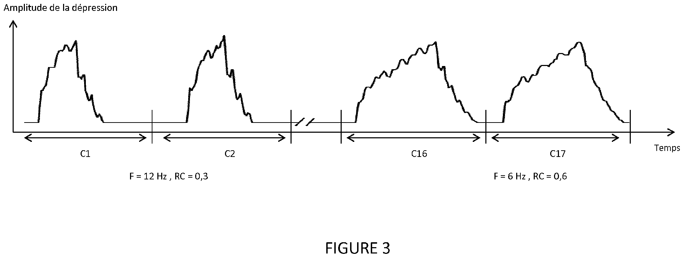

Par exemple, de manière illustrative et nullement limitative selon la figure 3 (C1 et C2), pour un rapport cyclique de 30%, le générateur de pression négative (6) va vider la chambre à vide (31) pendant 70% de 1/12^{ème} de seconde, et le patient (5) va être connecté à cette chambre à vide (31) pendant 30% de 1/12^{ème} de seconde. La période d'environ 83 ms d'une telle fréquence de 12 Hz se décompose donc pour le patient en une période d'aspiration (dépression) d'environ 24 ms et une période de pause d'environ 58 ms. Ceci provoque une dépression brève et forte donc un pouvoir liquéfiant plus fort par thixotropie.

Par exemple, de manière illustrative et nullement limitative selon la figure 3 (C16 et C17), pour un rapport cyclique de 60%, le générateur de pression négative (6) va vider la chambre à vide (31) pendant 40% de 1/6^{ème} de seconde, et le patient (5) va être connecté à cette chambre à vide (31) pendant 60% de 1/6^{ème} de seconde. La période d'environ 166 ms d'une telle fréquence de 6 Hz se décompose donc pour le patient en une période d'aspiration (dépression) d'environ 100 ms et une période de pause d'environ 66 ms. Ceci provoque une dépression moins forte et plus longue donc un pouvoir liquéfiant moins important mais un pouvoir de drainage plus fort par transmission de l'énergie cinétique entre l'air et le mucus sur un temps plus long c'est-à-dire 60% d'un cycle.

On notera que, sur la figure 3, le temps est coupé entre une première partie et une deuxième partie du traitement, mais surtout que l'échelle de temps représentée pour les deux premiers cycles (C1 C2) illustrés est environ deux fois plus rapide que celle des deux cycles ultérieurs (C16, C17) illustrés, puisque ces derniers n'apparaissent qu'environ deux moins plus lents que ces premiers, alors qu'ils sont environ quatre fois plus lents. D'autre part, comme représenté par exemple, de manière illustrative et nullement limitative selon la figure 3, une séance peut s'effectuer de la manière suivante : on effectue d'abord 15 cycles d'expiration passive à 12Hz avec un rapport cyclique de 30%, on obtient ainsi un fort pouvoir liquéfiant par thixotropie, puis on effectue 15 cycles d'expiration passive à 6 Hz avec un rapport cyclique de 70%, on obtient ainsi un drainage par transmission de l'énergie cinétique. Pendant les inspirations, le patient (5) se déconnecte du dispositif et inspire normalement.

Dans certains modes de réalisation, le dispositif comporte en outre un chronomètre en vue de déterminer la durée de chaque dépression appliquée au patient (5). Ledit chronomètre a pour avantage d'assister l'expiration du patient (5). Ainsi, il importe, pour qu'un cycle d'expiration soit efficace, que la stimulation s'opère pendant une durée minimale d'au moins cinq secondes et donc que l'expiration du patient (5) se maintienne pendant au moins un tel laps de temps. En effet, un faible temps de stimulation du patient (5) lors des phases expiratoires matérialise une faible capacité respiratoire et une descente en dépression trop rapide. Il en résulte une puissance trop importante du dispositif d'aspiration qui vide trop rapidement le volume pulmonaire du patient (5).

Dans certains modes de réalisation, le dispositif comporte en outre un calculateur permettant, sur la base de la valeur de dépression et de la durée de celle-ci mesurées à chaque cycle, de déterminer la valeur de dépression moyenne appliquée et la durée de stimulation moyenne pendant l'ensemble des cycles au patient (5). Ainsi, ledit calculateur enregistre dans sa mémoire à chaque commande de l'électrovanne (9), chaque valeur du signal provenant du capteur et représentatif de l'amplitude ou puissance de la dépression mesurée en instantané et le nombre de cycles de commande de l'électrovanne (9) pendant toute la durée (déterminée par un paramètre évolutif mémorisé, par l'appareil) du cycle d'expiration pour permettre au calculateur par un calcul utilisant les valeurs de puissance et le nombre de cycle de déterminer la valeur de dépression moyenne mesurée à chaque cycle, puis de la mémoriser pour ensuite déterminer la valeur de dépression moyenne appliquée pendant l'ensemble des cycles effectués par le patient (5) pendant la session de traitement. Le calculateur associé au capteur mesure également pendant les intervalles de temps où l'électrovanne (9) est fermée pour ne pas appliquer d'impulsion de dépression, l'évolution de la pression dans le poumon du patient (5) au cours de l'expiration et le temps nécessaire pour atteindre la valeur de pression correspondant à une expiration et mémorisée comme un seuil dans l'appareil. Ce temps correspondant à la durée d'un cycle d'expiration est mémorisé et utilisé par l'appareil. Cette valeur représentative de la durée du cycle d'expiration sera comparée à une première valeur de seuil, mémorisée par le calculateur qui représente une valeur de durée minimale pour faire ajuster par le calculateur la puissance de dépression générée par le dispositif lorsque la valeur représentative de la durée d'un cycle d'expiration chute en-dessous de la valeur minimale fixée. Une deuxième valeur de seuil qui représente une durée maximale de cycle d'expiration et mémorisée par le calculateur est utilisée par celui-ci en comparaison à la durée mesurée pour générer un signal de commande de l'augmentation de la dépression si la durée du cycle mesuré excède la deuxième valeur enregistrée.

Dans certains modes de réalisation, le dispositif comporte en outre un module de communication, permettant notamment l'envoi d'une alerte dès lors que la durée de stimulation moyenne marquera une diminution d'au moins 20% pour un patient (5). Ainsi, le module de communication est apte à établir une communication sans fil avec au moins un équipement distant selon un protocole de communication distinct donné. De la sorte, ledit module de communication permet la transmission de données aux professionnels de santé référents, notamment de la valeur de dépression moyenne ou instantané, de la durée de stimulation moyenne ou instantanée ou en accord de tout changement dans le traitement, notamment toute diminution marquée de l'une ou l'autre constituant ainsi un révélateur qui pourrait indiquer une dégradation de l'état du patient (5).

On pourra imaginer, par exemple, un algorithme de traitement du signal de mesure pour comparer celui ci à un seuil déterminé établi 20% en dessous de la valeur de consigne prévue par le système de programmation et l'envoi d'une alerte dès lors que la durée de stimulation moyenne marquera une diminution d'au moins 20% pour un patient (5). En effet, une telle baisse brutale constitue un signe d'une diminution des capacités respiratoires et musculaires du patient (5).

Dans certains modes de réalisation, le dispositif comporte en outre une carte microcontrôleur (10) qui, en fonction de la dépression mesurée par le capteur de pression (32), et les paramètres déterminés par l'utilisateur à l'intérieur d'une plage définie par un ensemble de modèles souhaitables mémorisés dans la mémoire associée au microcontrôleur (10), sera à même d'adapter la puissance appliquée au générateur de pression négative (6) de sorte d'obtenir une valeur recherchée de dépression appliquée au mucus trachéo-bronchique du patient (5). De préférence, la carte microcontrôleur (10) adaptera la puissance appliquée au générateur de pression négative (6) de sorte d'obtenir une dépression appliquée au mucus trachéo-bronchique du patient (5) comprise entre 40 et 100 millibars, de préférence entre 45 et 80 millibars, et de préférence encore entre 50 et 100 millibars. De la sorte, le traitement appliqué au patient (5) par le dispositif permettra d'obtenir l'efficacité optimale et de limiter l'impact de la variabilité interpatient. De même, la carte microcontrôleur (10) pourra également contrôler la puissance appliquée au générateur de pression négative (6) en fonction de la durée de stimulation mesurée. Le programme de fonctionnement du microcontroleur surveille la durée de stimulation et la compare à un seuil critique, pour baisser d'un pourcentage la puissance appliquée au générateur de pression négative (6). Ainsi dans le cas où la durée de stimulation passe sous le seuil critique de 5 secondes, la carte microcontrôleur (10) pourra baisser de 20% le signal représentatif de la consigne de puissance appliquée au générateur de pression négative (6), et donc la dépression appliquée au volume pulmonaire. Cette baisse permet d'abaisser la vitesse à laquelle est vidée le volume pulmonaire et donc d'augmenter la tolérance du patient (5). Dans le cas par contre où la durée de stimulation dépasse le seuil haut de 9 secondes, la carte microcontrôleur (10) pourra augmenter de 10% la puissance appliquée au générateur de pression négative (6), et donc la dépression appliquée au volume pulmonaire. Cette augmentation permet d'ajuster la performance de liquéfaction induite par la puissance de la dépression.

Dans certains modes de réalisation, le dispositif comporte une télécommande de sécurité de type homme mort, qui doit être activement actionné en permanence ou régulièrement réarmé par le patient (5), durant le traitement, pour que le dispositif fonctionne. A défaut, le dispositif s'arrête.

De préférence, une alimentation externe (11) alimente les différents composants du dispositif selon l'invention que sont le générateur de pression négative (6), le capteur de pression (32), le chronomètre et la carte microcontrôleur (10). Suite à la mise en route du dispositif selon l'invention, la carte microcontrôleur (10) va ajuster la puissance du générateur de pression négative (6) de sorte d'obtenir la valeur choisie de dépression (mbars).

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Un dispositif de stimulation (1) de l'air trachéo-bronchique d'un patient (5) souffrant d'un trouble ventilatoire obstructif et apte à modifier la rhéologie de son mucus trachéo-bronchique, qui comprend :
(i) un générateur de pression négative (6) ;
(ii) une interface physiologique (2) apte à interfacer le dispositif avec l'appareil respiratoire du patient (5) ;
(iii) un tuyau de raccordement (4) reliant l'interface physiologique (2) au générateur de pression négative (6) ; et
(iv) un circuit de contrôle capable de commander ledit générateur de pression négative (6), générant une expiration passive par l'application d'une succession d'alternances d'impulsions négatives de pression et de mise à l'atmosphère, avec une fréquence déterminée ;
**caractérisé en ce que** :
(v) ledit circuit de contrôle définit un premier paramètre, dit cycle d'expiration, correspondant à une période pendant laquelle lesdites impulsions négatives de pression sont appliquées au patient et un second paramètre, dit rapport cyclique, correspondant à un rapport entre la durée pendant laquelle une pression négative est appliquée au patient et la durée totale d'un cycle d'expiration ;
(vi) ledit circuit de contrôle réitéré un nombre défini de cycles d'expiration, en contrôlant ledit générateur avec une première fréquence de l'ordre de 10 à 15 Hz et un premier rapport cyclique de 0,2 à 0,7 pendant une première partie desdits cycles d'expiration, puis avec une deuxième fréquence de l'ordre de 4 à 7 Hz et un deuxième rapport cyclique de 0,5 à 0,8, pendant une deuxième partie des cycles d'expiration.

2. Le dispositif selon la revendication **1, caractérisé en ce que** le générateur de pression négative (6) comprend une pompe à vide (7) avec un débit supérieur à 20 L/min et une capacité de descente en vide d'au moins 200 mbars et un piège à son (8).

3. Le dispositif selon l'une des revendications **1** ou **2, caractérisé en ce que** le circuit de contrôle comprend une embase (3) pneumatique, une électrovanne (9) et un capteur de pression (32).

4. Le dispositif selon l'une quelconque des revendications **1** à **3, caractérisé en ce que** la première partie des cycles d'expiration comprend une fréquence de 12 Hz et un rapport cyclique de l'ordre de 0,3 et la deuxième partie des cycles comprend une fréquence de 6 Hz et un rapport cyclique de l'ordre de 0,6.

5. Le dispositif selon l'une quelconque des revendications **1** à **4, caractérisé en ce que** le circuit de contrôle est configuré pour adapter le cycle d'expiration préalablement choisi par l'opérateur ou le patient en fonction des résultats de durée de stimulation moyenne évaluées.

6. Le dispositif selon l'une quelconque des revendications **1** à **5, caractérisé en ce que** le circuit de contrôle est configuré pour recevoir des instructions en provenance de l'opérateur ou du patient afin de régler la puissance des dépressions au départ en fonction de leur tolérance.

7. Le dispositif selon l'une quelconque des revendications **1** à **6, caractérisé en ce qu'**il comprend en outre un chronomètre en vue de déterminer la durée des expirations pendant lesquelles les dépressions successives sont appliquées au patient (5).

8. Le dispositif selon l'une quelconque des revendications **1** à **7, caractérisé en ce qu'**il comprend en outre un calculateur permettant, sur la base de la valeur de dépression et de la durée de celle-ci mesurées à chaque cycle, de déterminer la valeur de dépression moyenne appliquée et la durée de stimulation moyenne pendant l'ensemble des cycles au patient (5).

9. Le dispositif selon l'une quelconque des revendications **1** à **8, caractérisé en ce qu'**il comprend en outre un module de communication, permettant notamment l'envoi d'une alerte dès lors que la durée de stimulation moyenne marquera une diminution d'au moins 20% pour un patient (5) et permet la communication vers une interface de contrôle des données comme une tablette tactile.

10. Le dispositif selon l'une quelconque des revendications **1** à **9, caractérisé en ce qu'**il comprend en outre une carte microcontrôleur (10) qui, en fonction de la dépression mesurée par le capteur de pression (32), est à même d'adapter la puissance appliquée au générateur de pression négative (6) de sorte d'obtenir une valeur recherchée de dépression appliquée au mucus trachéo-bronchique du patient (5).

11. Le dispositif selon la revendication **10, caractérisé en ce que** la carte microcontrôleur (10) est configurée pour adapter la puissance appliquée au générateur de pression négative (6) de sorte d'obtenir une dépression appliquée au mucus trachéo-bronchique du patient (5) comprise entre 40 et 100 millibars, de préférence entre 45 et 80 millibars, et de préférence encore entre 50 et 100 millibars.

12. Le dispositif selon l'une quelconque des revendications **10** ou **11, caractérisé en ce que** la carte microcontrôleur (10) est configurée pour également contrôler la puissance appliquée au générateur de pression négative (6) en fonction de la durée de stimulation mesurée.

13. Le dispositif selon la revendication **12, caractérisé en ce que** la carte microcontrôleur (10) est configurée pour baisser de 20% la puissance appliquée au générateur de pression négative (6) lorsque la durée de stimulation passe sous le seuil critique de 5 secondes lors d'une expiration.

14. Le dispositif selon la revendication **12, caractérisé en ce que** la carte microcontrôleur (10) permet d'augmenter de 10% la puissance appliquée au générateur de pression négative (6) dès lors que la durée de stimulation dépasse le seuil de 9 secondes lors d'une expiration.

## Patentansprüche

1. Vorrichtung zum Stimulieren (1) der tracheobronchialen Luft eines Patienten (5), der an einer obstruktiven Atemstörung leidet und dazu in der Lage ist, die Rheologie seines tracheobronchialen Schleims zu ändern, umfassend:
(i) einen Unterdruckgenerator (6);
(ii) eine physiologische Schnittstelle (2), die in der Lage ist, die Vorrichtung mit dem Atemgerät des Patienten (5) zu verbinden;
(iii) einen Verbindungsschlauch (4), der die physiologische Schnittstelle (2) mit dem Unterdruckgenerator (6) verbindet; und
(iv) eine Steuerschaltung, die dazu geeignet ist, den Unterdruckgenerator (6) zu steuern, der eine passive Ausatmung durch die Anwendung einer Folge von abwechselnden Unterdruck- und Entlüftungsimpulsen mit einer bestimmten Frequenz erzeugt;
**dadurch gekennzeichnet, dass**:
(v) die Steuerschaltung einen ersten Parameter, den sogenannten Ausatmungszyklus, definiert, der einem Zeitraum entspricht, während dem die Unterdruckimpulse auf den Patienten ausgeübt werden, und einen zweiten Parameter, das sogenannte Tastverhältnis, der einem Verhältnis zwischen der Dauer, während der ein Unterdruck auf den Patienten ausgeübt wird, und der Gesamtdauer eines Ausatmungszyklus entspricht,
(vi) die Steuerschaltung eine definierte Anzahl von Ausatmungszyklen wiederholt, indem sie den Generator mit einer ersten Frequenz in der Größenordnung von 10 bis 15 Hz und einem ersten Tastverhältnis von 0,2 bis 0,7 während eines ersten Teils der Ausatmungszyklen und dann mit einer zweiten Frequenz in der Größenordnung von 4 bis 7 Hz und einem zweiten Tastverhältnis von 0,5 bis 0,8 während eines zweiten Teils der Ausatmungszyklen steuert.

2. Vorrichtung nach Anspruch 1, **dadurch gekennzeichnet, dass** der Unterdruckgenerator (6) eine Vakuumpumpe (7) mit einer Durchflussrate von mehr als 20 L/min und einer Kapazität zur Vakuumabsenkung von mindestens 200 mbar sowie einen Geräuschdämpfer (8) umfasst.

3. Vorrichtung nach Anspruch 1 oder 2, **dadurch gekennzeichnet, dass** der Steuerkreis eine pneumatische Basis (3), ein Magnetventil (9) und einen Drucksensor (32) umfasst.

4. Vorrichtung nach einem der Ansprüche 1 bis 3, **dadurch gekennzeichnet, dass** der erste Teil der Ausatmungszyklen eine Frequenz von 12 Hz und ein Tastverhältnis in der Größenordnung von 0,3 umfasst und der zweite Teil der Zyklen eine Frequenz von 6 Hz und ein Tastverhältnis in der Größenordnung von 0,6 umfasst.

5. Vorrichtung nach einem der Ansprüche 1 bis 4, **dadurch gekennzeichnet, dass** die Steuerschaltung dazu konfiguriert ist, den zuvor vom Bediener oder Patienten gewählten Ausatmungszyklus in Abhängigkeit von den Ergebnissen der ausgewerteten durchschnittlichen Stimulationsdauer anzupassen.

6. Vorrichtung nach einem der Ansprüche 1 bis 5, **dadurch gekennzeichnet, dass** die Steuerschaltung dazu konfiguriert ist, Anweisungen vom Bediener oder vom Patienten zu empfangen, um die Leistung der Niederdrücke anfangs entsprechend ihrer Toleranz einzustellen.

7. Vorrichtung nach einem der Ansprüche 1 bis 6, **dadurch gekennzeichnet, dass** sie ferner einen Zeitmesser zwecks des Bestimmens der Dauer der Ausatmungen umfasst, während derer die aufeinanderfolgenden Niederdrücke auf den Patienten (5) ausgeübt werden.

8. Vorrichtung nach einem der Ansprüche 1 bis 7, **dadurch gekennzeichnet, dass** sie ferner einen Rechner umfasst, der es ermöglicht, auf der Grundlage des bei jedem Zyklus gemessenen Niederdruckwerts und der Dauer desselben den durchschnittlich angewandten Niederdruckwert und die durchschnittliche Stimulationsdauer während aller Zyklen am Patienten (5) zu bestimmen.

9. Vorrichtung nach einem der Ansprüche 1 bis 8, **dadurch gekennzeichnet, dass** sie ferner ein Kommunikationsmodul umfasst, das insbesondere das Senden einer Warnung ermöglicht, wenn die durchschnittliche Stimulationsdauer für einen Patienten (5) einen Rückgang von mindestens 20 % markiert, und die Kommunikation mit einer Datensteuerungsschnittstelle wie einem Touchscreen-Tablet ermöglicht.

10. Vorrichtung nach einem der Ansprüche 1 bis 9, **dadurch gekennzeichnet, dass** sie ferner eine Mikrocontroller-Karte (10) umfasst, die in der Lage ist, in Abhängigkeit von dem durch den Drucksensor (32) gemessenen Niederdruck die an den Unterdruckgenerator (6) angelegte Leistung anzupassen, um einen gewünschten Wert des auf den tracheobronchialen Schleim des Patienten (5) ausgeübten Niederdrucks zu erhalten.

11. Vorrichtung nach Anspruch 10, **dadurch gekennzeichnet, dass** die Mikrocontroller-Karte (10) dazu konfiguriert ist, die an den Unterdruckgenerator (6) angelegte Leistung anzupassen, um einen auf den tracheobronchialen Schleim des Patienten (5) ausgeübten Niederdruck zwischen 40 und 100 Millibar, vorzugsweise zwischen 45 und 80 Millibar und noch bevorzugter zwischen 50 und 100 Millibar, zu erhalten.

12. Vorrichtung nach einem der Ansprüche 10 oder 11, **dadurch gekennzeichnet, dass** die Mikrocontroller-Karte (10) dazu konfiguriert ist, ebenfalls die an den Unterdruckgenerator (6) angelegte Leistung in Abhängigkeit von der gemessenen Stimulationsdauer zu steuern.

13. Vorrichtung nach Anspruch 12, **dadurch gekennzeichnet, dass** die Mikrocontroller-Karte (10) dazu konfiguriert ist, die an den Unterdruckgenerator (6) angelegte Leistung um 20 % zu senken, sobald die Stimulationsdauer während einer Ausatmung den kritischen Schwellenwert von 5 Sekunden unterschreitet.

14. Vorrichtung nach Anspruch 12, **dadurch gekennzeichnet, dass** die Mikrocontroller-Karte (10) es ermöglicht, die an den Unterdruckgenerator (6) angelegte Leistung um 10 % zu erhöhen, wenn die Stimulationsdauer während einer Ausatmung den Schwellenwert von 9 Sekunden überschreitet.

## Claims

1. A device (1) for stimulating the tracheobronchial air of a patient (5) suffering from an obstructive ventilatory disorder and able to modify the rheology of his tracheobronchial mucus, which comprises:
(i) a negative pressure generator (6);
(ii) a physiological interface (2) able to interface the device with the patient's respiratory apparatus (5);
(iii) a connecting pipe (4) connecting the physiological interface (2) to the negative pressure generator (6); and
(iv) a control circuit capable of controlling said negative pressure generator (6), generating a passive expiration by applying a succession of alternating negative pressure and venting impulses, with a determined frequency;
**characterized in that**:
(v) said control circuit defines a first parameter, named expiration cycle, corresponding to a period during which said negative pressure impulses are applied to the patient, and a second parameter, named duty cycle, corresponding to a ratio between the time during which negative pressure is applied to the patient and the total duration of an expiration cycle;
(vi) said control circuit repeats a defined number of exhalation cycles, controlling said generator with a first frequency of the order of 10 to 15 Hz and a first duty cycle of 0.2 to 0.7 during a first part of said expiration cycles, then with a second frequency of the order of 4 to 7 Hz and a second duty cycle of 0.5 to 0.8, during a second part of the expiration cycles.

2. The device according to claim 1, **characterized in that** the negative pressure generator (6) comprises a vacuum pump (7) with a flow rate greater than 20 L/min and a vacuum descent capacity of at least 200 mbar and a sound trap (8).

3. The device according to claim 1 or 2, **characterized in that** the control circuit comprises a pneumatic base (3), a solenoid valve (9) and a pressure sensor (32).

4. The device according to any one of claims 1 to 3, **characterized in that** the first part of an expiration cycle comprises a frequency of 12 Hz and a duty cycle of the order of 0.3 and the second part. a cycle comprises a frequency of 6 Hz and a duty cycle of the order of 0.6.

5. The device according to any one of claims 1 to 4, **characterized in that** the control circuit is configured to adapt the expiration cycle previously chosen by the operator or the patient according to the results of evaluated average stimulation duration.

6. The device according to any one of claims 1 to 5, **characterized in that** the control circuit is configured to receive instructions from the operator or patient to adjust the power of the departing depressions according to their tolerance.

7. The device according to any one of claims 1 to 6, **characterized in that** it further comprises a stopwatch for determining the duration of expirations during which the successive depressions are applied to the patient (5).

8. The device according to any one of claims 1 to 7, **characterized in that** it further comprises a calculator making it possible, on the basis of the depression value and duration measured at each cycle, to determine the average value of depression applied and the average duration of stimulation during all cycles to the patient (5).

9. The device according to any one of claims 1 to 8, **characterized in that** it further comprises a communication module, in particular allowing the sending of an alert when the average duration of stimulation will mark a decrease of at least 20% for a patient (5) and allows communication to a data control interface as a touch pad.

10. The device according to any one of claims 1 to 9, **characterized in that** it further comprises a microcontroller card (10) which, depending on the depression measured by the pressure sensor (32), is able to adapt the power applied to the negative pressure generator (6) to obtain a desired value of depression applied to the tracheobronchial mucus of the patient (5).

11. The device according to claim 10, **characterized in that** the microcontroller card (10) is configured to adapt the power applied to the negative pressure generator (6) to obtain a depression applied to the tracheobronchial mucus of the patient (5) of between 40 and 100 millibars, preferably between 45 and 80 millibars, preferably between 50 and 100 millibars.

12. The device according to any one of claims 10 or 11, **characterized in that** the microcontroller card (10) is configured to also control the power applied to the negative pressure generator (6) as a function of the measured duration of stimulation.

13. The device according to claim 12, **characterized in that** the microcontroller card (10) is configured to lower by 20% the power applied to the negative pressure generator (6) as soon as the duration of stimulation goes below the critical threshold of 5 seconds during an expiration.

14. The device according to claim 12, **characterized in that** the microcontroller card (10) makes it possible to increase by 10% the power applied to the negative pressure generator (6) when the duration of stimulation exceeds the threshold of 9 seconds during an expiration.
